# EUROPEAN PATENT APPLICATION

(11) **EP 0 955 013 A1**
(43) Date of publication of application: **10.11.1999**
(21) Application number: 99401087.4
(22) Date of filing: 04.05.1999
(51) Int. Cl.: A61B 17/74, A61F 2/36

(54) **Elément destiné à stabiliser des fractures trochantériennes et pince passe-cáble pour sa mise en place**

(30) Priority: 04.05.1998 FR 9805622
(71) Applicant: Porte, Michel, 93150 Le Blanc-Mesnil (FR); Cirotteau, Yves, 75007 Paris (FR)
(72) Inventor: Porte, Michel, 93150 Le Blanc-Mesnil (FR); Cirotteau, Yves, 75007 Paris (FR)
(74) Representative: Obolensky, Michel

(57) **Abrégé**

Elément de prothèse de la hanche, comprenant un corps (8) destiné à être fixé au fémur (2) et un élément cervical (12) prolongeant le corps et faisant avec celui-ci un angle prédéterminé d'orientation vers la tête du fémur, caractérisé en ce qu'il comporte au moins un lien (26,32) de stabilisation en forme de boucle de remise en place et de maintien du trochanter (3,4) fracturé correspondant, les extrémités du lien traversant des orifices (20,22) ménagés dans le corps (8) de l'élément de prothèse et portant des butées (30,36) d'immobilisation par rapport audit corps. Aussi, pince passe-câble (80) destinée à permettre la mise en place des liens (26,32).

## Description

La présente invention est relative aux éléments de prothèse de hanche et se rapporte plus particulièrement aux éléments de prothèse à appui cortical externe destinés à réduire et stabiliser des fractures trochantériennes d'extrémité supérieure du fémur.

Parmi les éléments de prothèses à appui cortical externe, on peut citer:
- les clous plaques
- les vis plaques qu'elles soient dynamiques ou stabilisées
- les lames plaques
- certaines prothèses de hanche.

Tous ces éléments de prothèses ont pour point commun, de comporter une plaque destinée à être fixée à l'aide de vis à l'extérieur du fémur fracturé, cette plaque étant prolongée en direction de la tête du fémur par un clou, une vis, une plaque ou une portion de sphère.

Les fractures du col du fémur se produisent dans un grand nombre de cas chez des sujets âgés, en particulier chez les femmes souffrant d'ostéoporose.

L'ostéoporose provoque dans la région du col du fémur, une destruction de la masse osseuse au point qu'il ne peut rester que 5% de cette masse osseuse dans cette région.

Il s'ensuit que dans la région du col du fémur, l'os devient creux de sorte que s'il y a fracture, les parois de la cavité formée par disparition du tissu osseux présentent une résistance mécanique dans certains cas insuffisante pour recevoir des vis ou des clous de la plaque de l'élément de prothèse.

Le problème qui se pose alors pour le chirurgien est de deux natures :
a) il faut combler la cavité formée par la disparition du tissu osseux, à l'aide d'un matériau biologiquement acceptable par le milieu dans lequel il est implanté;
b) il faut permettre au patient de retrouver après la pose d'une prothèse, une autonomie pratiquement normale dans un délai le plus court possible.

La diaphyse fémorale comporte deux saillies osseuses ayant un rôle essentiel pour le fonctionnement de l'articulation de la hanche.

Il s'agit du grand trochanter et du petit trochanter auxquels sont respectivement reliés les extrémités du muscle moyen-fessier et du muscle psoas iliaque.

Dans certaines fractures dites trochantériennes, le grand trochanter, le petit trochanter et quelquefois les deux, se détachent de la diaphyse et sont entraînés par les muscles moyen-fessier auxquels ils sont normalement reliés.

Lors du traitement de fractures de ce type, on s'intéressait jusqu'à présent à la stabilisation du fragment principal et on négligeait les grosses tubérosités telles que les trochanters dont la remise en place est pourtant essentielle pour permettre au patient de retrouver rapidement sa mobilité et de ce fait, son autonomie de mouvements.

L'invention vise à créer un élément de prothèse qui tout en étant d'une construction très voisine de celle des éléments de prothèses classiques permette d'améliorer considérablement le traitement des fractures trochantériennes.

Elle a donc pour objet un élément de prothèse de la hanche, comprenant un corps destiné à être fixé au fémur et un élément cervical prolongeant le corps et faisant avec celui-ci un angle prédéterminé d'orientation vers la tête du fémur, caractérisé en ce qu'il comporte au moins un lien en forme de boucle de remise en place et de maintien du trochanter fracturé correspondant, les extrémités du lien traversant des orifices ménagés dans le corps de l'élément de prothèse et portant des butées d'immobilisation par rapport audit corps.

L'invention sera mieux comprise à l'aide de la description qui va suivre, donnée uniquement à titre d'exemple et faite en se référant aux dessins annexés, sur lesquels :
- la Fig.1 est une vue schématique en perspective d'une articulation de la hanche dans laquelle est monté un élément de prothèse suivant l'invention en forme de clou plaque à appui cortical externe, muni de liens en forme de boucles de remise en place et de maintien du grand trochanter et du petit trochanter suivant l'invention;
- la Fig.2 est une vue en perspective à plus grande échelle montrant un détail de l'élément de prothèse de la figure 1;
- la Fig.3 est une vue en perspective d'un élément de prothèse en forme de vis-plaque suivant l'invention;
- la Fig.4 est une vue en perspective et en coupe partielle d'une prothèse à appui cortical externe suivant l'invention;
- la Fig.5 est une vue en perspective d'une pince passe-câble destinée à la mise en place de liens de remise en place et de maintien des trochanters; et
- les Figs.6 à 9 sont des vues schématiques représentant le procédé de stabilisation des tubérosités trochantériennes mettant en oeuvre un élément de prothèse suivant l'invention.

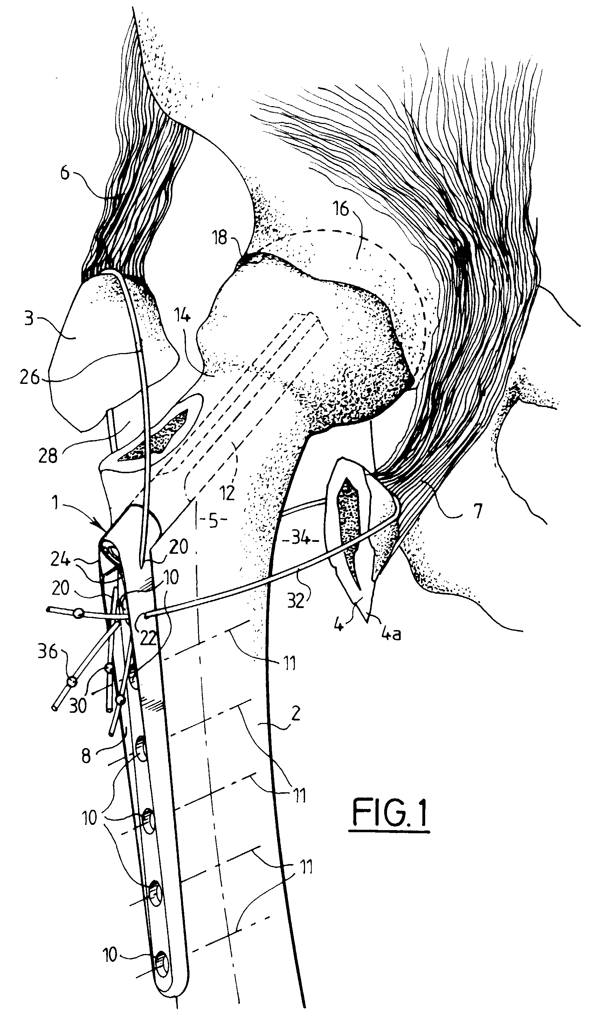

Sur la figure 1, on a représenté un élément de prothèse suivant l'invention constitué par un clou-plaque désigné par le numéro de référence 1 monté dans un fémur 2 dont le grand trochanter 3 et le petit trochanter 4 sont fracturés et sont détachés de la diaphyse 5 sous l'effet de la rétraction du muscle moyen-fessier 6 et du muscle psoas iliaque 7 auxquels ils sont respectivement fixés.

Le clou-plaque 1 comporte un corps ou plaque 8 d'appui cortical externe appliqué contre la face externe de paroi latérale du fémur 2 et pourvu de trous 10 dans lesquels sont engagées des vis de fixation 11 de la plaque 8 au fémur, matérialisées par leurs axes.

A son extrémité supérieure, la plaque 8 est prolongée par une tige ou clou 12 engagée dans le col 14 et dans la tête 16 du fémur.

Le clou 12 fait avec la plaque 8, un angle d'une valeur telle qu'il est dirigé à peu près vers le centre de la tête du fémur 18. Cette valeur peut varier de 5° en 5° de 110° à 150°.

Le clou-plaque 1 est pourvu dans la région de la jonction du clou 12 avec la plaque 8 de perçages ou orifices 20,22 ménagés dans les bords longitudinaux opposés 24 de la plaque.

Dans les deux perçages 20 est engagé un lien souple et résistant, par exemple un câble d'acier 26, qui forme une boucle 28 passant dans l'extrémité du muscle fessier, derrière la tubérosité du grand trochanter fracturé 3 et destiné à la remise en place et au maintien du grand trochanter à peu près à son emplacement initial sur la diaphyse 5.

Le câble peut également être réalisé en polymère bio-compatible, ou en matériau bio-résorbable.

Le câble 26 est d'abord passé dans un outil approprié dont un exemple sera décrit par la suite, puis dans le tendon du muscle moyen-fessier 6 au-dessus du grand trochanter.

L'outil est ensuite retiré, le câble restant en place. Le câble est alors inséré dans les perçages 20 du clou-plaque.

Le chirurgien exerce alors une traction sur les deux extrémités du câble 26 de manière à déplacer la boucle 26 ainsi formée et à ramener le grand trochanter 3 à peu près dans sa position correcte par rapport à la diaphyse 5.

Cette position étant atteinte, les extrémités du câble 26 reçoivent par sertissage des éléments de butée tels que des billes 30 de section plus importante que celle des perçages 20 correspondants et les tronçons d'extrémité du câble 26 s'étendant au-delà des billes de butée sont sectionnés.

Les orientations des orifices ou perçages 20 sont déterminées afin de donner au câble 26 l'orientation la plus favorable possible pour lui permettre d'atteindre le grand trochanter 3 qui s'est détaché de la diaphyse et qui est porté par le muscle moyen-fessier 6 rétracté.

La remise en place du petit trochanter 4 est assurée de façon analogue à l'aide du câble 32 qui est successivement engagé dans le passe-câble mis en place et qui est passé au-dessus du petit trochanter fracturé 4.

Après avoir retiré le passe-câble, on engage le câble dans les perçages 22 de la plaque 8.

Par traction sur la boucle 34 constituée par le câble 32, le petit trochanter 4 est remis en place et les deux brins du câble 32 situés au-delà des perçages 22 par rapport à la boucle 34, sont munis par sertissage de butées 36 en forme de billes et leurs extrémités s'étendant au-delà de ces butées sont sectionnées.

Les orientations des orifices 22 sont également déterminées afin de donner au câble 32 l'orientation la plus favorable possible pour lui permettre d'atteindre le petit trochanter 4.

Ainsi, le grand trochanter 3 et le petit trochanter 4 se trouvent dans des conditions optimales pour se ressouder à la diaphyse.

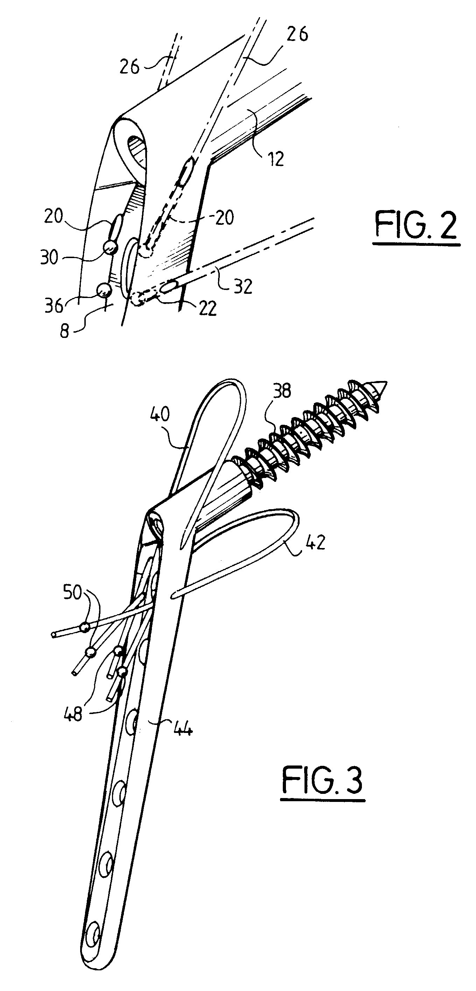

La figure 2 montre clairement les orientations des perçages 20 et 22, ménagées dans les bords de la plaque 8 du clou-plaque de la figure 1.

La figure 3 est une vue en perspective d'une vis-plaque suivant l'invention.

Cette vis plaque est en tous points semblable au clou-plaque représenté aux figures 1 et 2 à l'exception du fait qu'elle comporte une vis 38 au lieu du clou 12.

En ce qui concerne les liens souples 40 et 42 de remise en place et de maintien du grand trochanter et du petit trochanter, ils sont disposés par rapport à la plaque 44 de la vis-plaque de la même façon que les liens 26,32 correspondants que comporte le clou-plaque décrit en référence aux figures 1 et 2. Ils traversent la plaque 44 par des orifices 46,47 respectifs.

Des butées en forme de billes 48,50 sont associées aux extrémités des liens 40,42 réalisés sous forme de câbles de la façon décrite en référence aux figures 1 et 2.

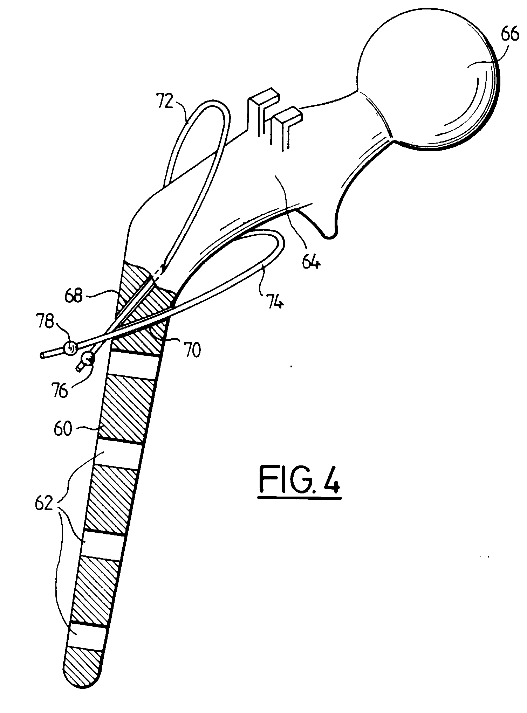

La prothèse à appui cortical externe représentée à la figure 4 comporte une plaque 60 pourvue de trous 62 de réception de vis de fixation au fémur.

La plaque 60 est prolongée par une partie 64 formant col terminée par une boule 66 formant la tête de la prothèse destinée à être reçue dans la cavité cotyloïde de l'os iliaque.

Dans la partie de la jonction de la plaque 60 et de l'élément formant col 64, sont ménagés des perçages inclinés 68,70 de réception de liens souples 72,74 formés par des câbles, de remise en place et de maintien respectivement du grand trochanter et du petit trochanter d'une manière déjà décrite en référence aux figures 1 et 2.

Aux extrémités libres des câbles 72 et 74 sont montées des butées respectives 76,78 de section supérieure à celle des perçages 68,70 et destinées à immobiliser les câbles 72,74 correspondants par sertissage sur ceux-ci après qu'ils aient été placés par le chirurgien dans leurs positions de maintien en place des trochanters.

L'invention a été décrite comme étant applicable aux clous-plaques, aux vis-plaques et aux prothèses à appui cortical externe.

Elle est également applicable aux lames-plaques.

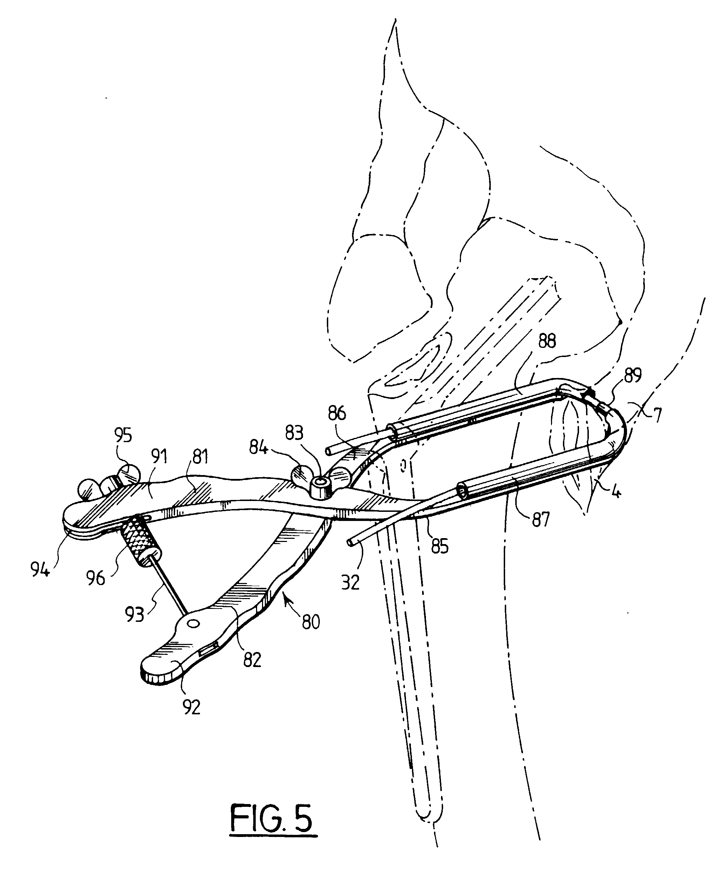

Sur la figure 5, on a représenté en perspective une pince passe-câble destinée à permettre la mise en place des câbles ou liens de remise en place et de maintien des trochanters fracturés.

Cette pince qui porte la référence générale 80 comporte deux branches 81,82 articulées entre elles par un axe d'articulation 83 et assemblées de façon démontable par exemple au moyen d'un écrou à oreilles 84 vissé sur une extrémité filetée de l'axe d'articulation 83.

Les mâchoires 85,86 des branches 81,82 comportent chacune un tube passe-câble 87,88 recourbé à son extrémité opposée à l'articulation des branches.

L'un des tubes passe-câble 87 comporte un embout tubulaire mâle 89 destiné à être engagé dans l'extrémité recourbée de l'autre tube passe-câble 88 pour former un canal continu permettant le passage sans obstacle d'un câble tel que le câble 32 lorsque les extrémités des mâchoires 87,88 de la pince sont engagées dans les tissus du muscle psoas iliaque 7 solidaire de la tubérosité du petit trochanter 4 représenté comme le reste de l'articulation du fémur et l'élément de prothèse 1, en trait mixte sur la figure 5.

Les poignées 91,92 des branches 81,82 de la pince passe-câble sont réunies entre elles par une tige 93 articulée sur l'une des poignées 92 et engagée dans une fente 94 ménagée dans l'autre poignée 91 de la pince.

La tige 93 est filetée et une vis de serrage 95 est montée sur l'extrémité libre de la tige 93 à l'extérieur de la poignée 94. A l'intérieur de la poignée 91, la tige 93 porte une butée 96 de réglage du degré de serrage de la pince.

La remise en place ou stabilisation des tubérosités des trochanters est réalisée da la manière suivante.

Les tubérosités sont stabilisées en dernier lieu après la mise en place de l'élément de prothèse tel que le clou-plaque 1 de la figure 1.

Ce temps opératoire se déroule donc après l'ostéosynthèse de la fracture trochantérienne par un clou-plaque, une vis-plaque ou autre.

Il peut être nécessaire de stabiliser le petit trochanter dans trois cas.
a) le petit trochanter est resté en place mais il existe un trait de fracture partiel ou total, habituellement vertical qui isole le petit trochanter du fût diaphysaire, ce qui entraîne un risque de déplacement secondaire du petit trochanter.
b) le petit trochanter est détaché de la corticale et prolongé vers le bas par une écaille de taille variable.

Cette situation est clairement représentée à la figure 1 sur laquelle on voit le petit trochanter 4 détaché de la corticale et pourvu d'une écaille 4a.

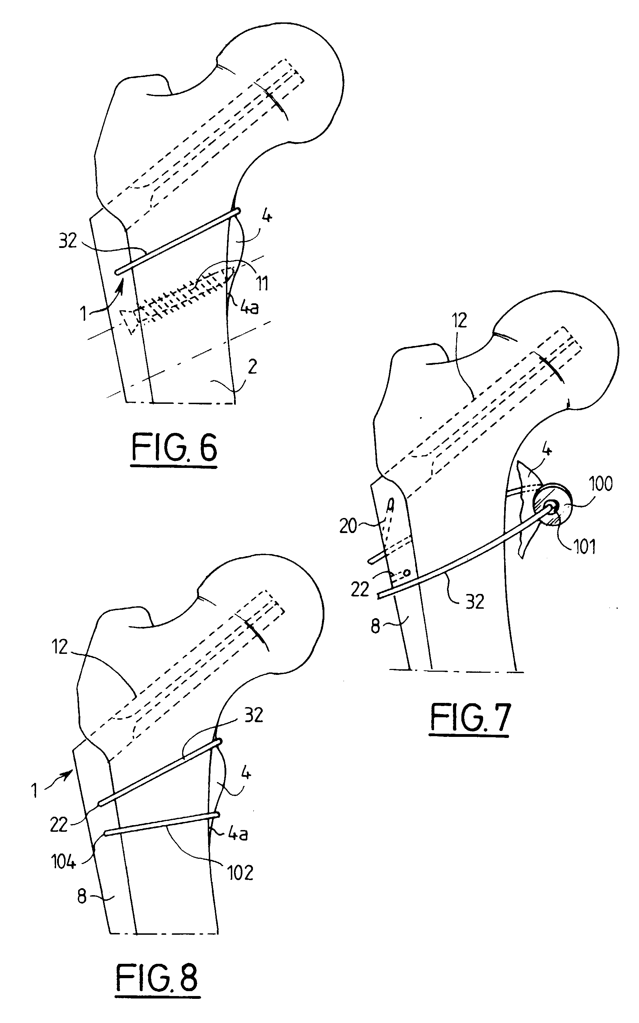

Comme représenté sur le schéma de la figure 6, le petit trochanter 4 est abaissé à l'aide du câble 32 puis l'écaille 4a est stabilisée à l'aide d'un cerclage au moyen de ce câble.

Si l'écaille 4a a une étendue verticale suffisante, elle peut également être stabilisée à sa partie inférieure par une des vis 11 de fixation du clou-plaque sur la corticale externe du fémur 2 (figure 6).

Une stabilisation par cerclage analogue à celle de la figure 6 est également réalisée pour stabiliser un trochanter en place, mais il y a un risque de déplacement secondaire du fait de l'existence d'un trait de fracture.
c) le petit trochanter est détaché et est venu se placer sous le col, contre le pôle inférieur de la tête fémorale.

Il est en outre parfois fracturé.

En vue de la stabilisation du petit trochanter se trouvant dans cet état, on utilise la pince passe-câble représentée à la figure 5 de la façon suivante :
- on fait d'abord passer la branche antérieure 81 de la pince en avant du col, au-dessus du petit trochanter 4,
- on fait ensuite passer la branche postérieure 82 de la pince en arrière du col, au-dessus du petit trochanter 4,
- on solidarise les branches 81 et 82 au moyen de l'axe d'articulation 83 et de l'écrou 84.

A l'aide de l'écrou de serrage 95, on rapproche les deux poignées 91, 92 et de ce fait, leurs extrémités des mâchoires 85,86 de la pince afin qu'elles traversent le tendon blanc du muscle psoas-iliaque 7 représenté en trait mixte.

L'embout tubulaire mâle 89 du tube passe-câble 87 s'engage alors dans l'extrémité du tube passe-câble 88 et établit un canal continu pour le passage d'un câble.
- on fait passer le câble 32 dans le conduit ainsi constitué,
- on sépare les deux branches 81,82 de la pince par dévissage de l'écrou 84 et retrait de la tige 93 associée à l'écrou de serrage 95 hors de la fente 94 de la poignée 91 de la pince;
- on retire successivement la branche antérieure 81 et la branche postérieure 82 de la pince en maintenant le câble 90 en place,
- on obtient alors la situation représentée à la figure 1 dans laquelle le câble 32 est engagé dans le muscle psoas-iliaque derrière le petit trochanter 4 et ses extrémités libres traversent la plaque 8 par les perçages 22.

Pour réduire le petit trochanter, on combine deux manoeuvres :
- on abaisse le petit trochanter lorsqu'il est en position haute,
- on l'amène en arrière puisqu'il est déplacé en avant et en dedans.

Cette réduction retend également la capsule antérieure.

Une fois réduit, le petit trochanter 4 se trouve dans l'état représenté à la figure 6.

Comme représenté à la figure 7, le petit trochanter a subi lors de la fracture, un déplacement latéral sous l'effet de la rétraction du muscle psoas-iliaque.

En l'occurrence, le déplacement latéral a eu lieu vers l'avant du col.

Afin de repositionner correctement le petit trochanter 4, après avoir fait passer le câble 32 dans le muscle psoas-iliaque comme décrit précédemment en référence à la figure 5, on engage sur le brin antérieur du câble, une rondelle métallique 100 et une bille de butée 101 pour la rondelle.

A l'aide d'une pince à sertir, on fixe la bille 101 sur le câble 32.

On déplace alors doucement la rondelle 100 au contact du trochanter 4 en exerçant une traction sur le brin postérieur du câble 32.

On fait ensuite passer les deux brins du câble 32 dans les passages 22 percés dans la plaque 8 du clou-plaque.

On exerce un traction sur les deux brins du câble pour amener le petit trochanter à sa place.

On stabilise l'ensemble à l'aide des deux billes telles que les billes 36 (Fig.1) serties sur le câble 32.

En fonction de la longueur de l'écaille interne 4a, on peut être amené à stabiliser le petit trochanter avec deux cerclages ou plus.

Sur la figure 8, le petit trochanter 4 a été stabilisé avec deux cerclages constitués l'un du câble 32 et l'autre, d'un câble supplémentaire 102 mis en place et immobilisé dans les mêmes conditions que le câble 32 et traversant des orifices 104 ménagés dans la plaque 8 au-dessous des passages 22 pour le câble 32.

La stabilisation du grand trochanter est réalisée selon l'invention à l'aide de la pince passe-câble, de la même manière que celle du petit trochanter, en faisant passer la branche postérieure de la pince passe-câble en avant ou à l'intérieur du tendon blanc du moyen-fessier.

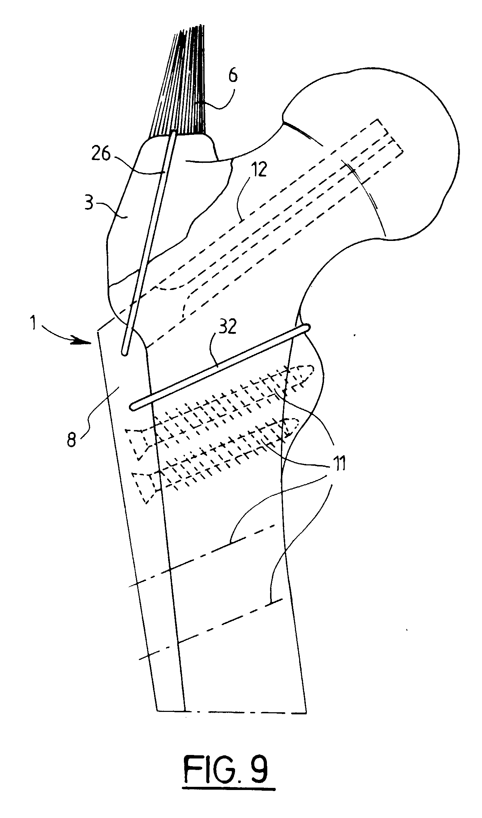

La figure 9 représente schématiquement un montage terminé d'un clou-plaque 1 sur l'invention, le grand trochanter 3 étant réduit à l'aide du câble 26, immobilisé par rapport à la plaque 8 par les billes serties 30 (Fig.1) et coupé à la longueur voulue, tandis que le petit trochanter 4 est réduit à l'aide du câble 32.

On voit que grâce à l'agencement qui vient d'être décrit, on parvient à réduire des fractures trochantériennes en remettant en place les trochanters, ce qui permet au patient de recouvrer rapidement sa stabilité lors de l'appui monopodal et sa force musculaire lors de la montée ou de la descente des escaliers.

## Revendications

1. Elément de prothèse de la hanche, comprenant un corps (8;44;60) destiné à être fixé au fémur (2) et un élément cervical (12;38;64) prolongeant le corps et faisant avec celui-ci un angle prédéterminé d'orientation vers la tête du fémur, caractérisé en ce qu'il comporte au moins un lien (26;32; 40,42;72,74;32,102) en forme de boucle de remise en place et de maintien du trochanter (3,4) fracturé correspondant, les extrémités du lien traversant des orifices (20,22;46,47;68,70;22,104) ménagés dans le corps (8;44;60) de l'élément de prothèse et portant des butées (30,36;48,50;76,78) d'immobilisation par rapport audit corps.

2. Elément de prothèse suivant la revendication 1, caractérisé en ce que chacun desdits liens (26,32;40,42; 72,74) est un câble en acier ou en polymère bio-compatible, ou en matériau bio-résorbable.

3. Elément de prothèse suivant la revendication 2, caractérisé en ce que les orientations des orifices (20,22;46,47;68,70) ménagés dans le corps (8;44;60) sont déterminées afin de donner aux câbles (26,32;40,42;72,74) les orientations les plus favorables possibles pour leur permettre d'atteindre les trochanters (3,4) correspondants lorsqu'ils se sont détachés de la diaphyse et sont portés par les muscles correspondants qui se sont rétractés.

4. Elément de prothèse suivant l'une des revendications 2 et 3, caractérisé en ce que les butées (30,36;48,50; 76,78) d'immobilisation des câbles (26,32;40,42;72,74) par rapport au corps (8;44;60) de l'élément de prothèse sont des billes fixées sur les câbles par sertissage.

5. Elément de prothèse suivant l'une quelconque des revendications 1 à 4, caractérisé en ce qu'il comporte en outre une rondelle métallique (100) associée à une bille de butée (101) fixée sur un câble à proximité d'un trochanter (4) ayant subi un déplacement latéral et derrière lequel le câble a été passé, ladite rondelle étant montée sur un brin du câble (32) situé d'un côté du trochanter et formant un organe de déplacement du trochanter par contact avec celui-ci sous l'action d'une traction exercée sur l'autre brin du câble (32).

6. Pince passe-câble pour la pose des câbles de l'élément de prothèse suivant l'une des revendications 2 à 5, caractérisée en ce qu'elle comporte deux branches (81,82) articulées par des moyens d'articulation démontables (83,84), les branches (81,82) comprenant des tubes passe-câble (87,88) respectifs recourbés à leurs extrémités opposées à l'articulation des branches.

7. Pince passe-câble suivant la revendication 6, caractérisée en ce qu'un des tubes passe-câble (87) comporte à son extrémité recourbée un embout tubulaire mâle (89) destiné à être engagé dans l'extrémité recourbée de l'autre tube passe-câble (88) pour former un canal continu permettant le passage sans obstacle d'un câble (32) lorsque les extrémités des mâchoires (85,86) de la pince sont engagées dans les tissus d'un muscle associé au trochanter (3,4) à réduire.

8. Pince passe-câble suivant l'une des revendications 6 et 7, caractérisée en ce qu'elle comporte une tige (93) articulée sur une poignée (92) de la pince et engagée dans une fente (94) ménagée dans l'autre poignée (91) de la pince, un écrou (95) de serrage de la pince étant porté par une extrémité filetée de la tige (93) à l'extérieur de ladite autre poignée (91).
